(19) Europäisches Patentamt / European Patent Office / Office européen des brevets

(11) **EP 4 555 932 A1**

(12) **EUROPEAN PATENT APPLICATION**
published in accordance with Art. 153(4) EPC

(43) Date of publication:
**21.05.2025 Bulletin 2025/21**

(21) Application number: **23867562.3**

(22) Date of filing: **20.09.2023**

(51) International Patent Classification (IPC):
**A61B 5/346** (2021.01)      **A61B 5/366** (2021.01)

(86) International application number:
**PCT/CN2023/120071**

(87) International publication number:
**WO 2024/061275 (28.03.2024 Gazette 2024/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **20.09.2022 CN 202211148197**

(71) Applicant: **Hangzhou Gray Dynamics Innovation
Ltd
Hangzhou, Zhejiang 311121 (CN)**

(72) Inventors:
• **JIANG, Ning**
  **Hangzhou, Zhejiang 311121 (CN)**
• **NEIJISI, Haidarui benni**
  **Hangzhou, Zhejiang 311121 (CN)**
• **HE, Jiayuan**
  **Hangzhou, Zhejiang 311121 (CN)**

(74) Representative: **Bandpay & Greuter
11, rue Christophe Colomb
75008 Paris (FR)**

(54) **SURFACE ELECTROPHYSIOLOGICAL SIGNAL PROCESSING METHOD FOR ELIMINATING SIGNAL PHASE SHIFT AND SYSTEM**

(57)     The present disclosure belongs to the technical field of medical devices, and specifically relates to a method and a system for processing the surface electrophysiological signals to eliminate signal phase shifts. The method of the present disclosure includes steps of: acquiring surface electrophysiological signals from a human skin surface; processing surface electrophysiological signal data using a zero-phase filter based on a stationary wavelet transform, to obtain wavelet detail coefficients and approximate coefficients; detecting a QRS wave complex for each layer of wavelet detail coefficients, and obtaining a heartbeat detection result from each layer of wavelet detail coefficients by a weighted voting scheme, such that source fusion is realized. The present disclosure further provides a system and a device applying the above method. The present disclosure is capable of accurately extracting electrophysiological signal components of the fixed time-domain characteristic waveforms, such as QRS wave complex in ECG signals, in a high-noise environment, without causing distortion of the waveforms due to the phase shifts in the extracted signals. It is highly applicable to application scenarios such as obtaining a long-duration dynamic electrocardiogram from a nontraditional site such as on the upper arm, behind the ear, or inside the ear, and has good application prospects.

FIG. 1

## Description

### TECHNICAL FIELD

The present disclosure belongs to the technical field of medical devices, and particularly, to a method and a system for processing the surface electrophysiological signals to eliminate the signal phase shifts.

### BACKGROUND

Cardiovascular disease is the number one cause of death worldwide. Approximately 50% of deaths are usually caused by cardiac arrhythmias. Accurate recording and detecting various cardiac arrhythmias are critical to preventing death from these conditions. Heartbeat detection is essential to determine heart rate and associated arrhythmias. Electrocardiogram (ECG) is the most widely used clinical tool that provides a graphic representation of the electrical activity of the heart detected from the body surface. Most importantly, the ECG waveform is the QRS complex (representing ventricular depolarization), which plays a fundamental role in heartbeat detection. Therefore, accurate detection of the QRS wave complex is the most important step before any ECG analysis. One of the many methods for detecting QRS wave complex in ECG is the discrete wavelet transform (DWT). DWT satisfies the law of conservation of energy, enables perfect reconstruction of the signals, and provides efficient computation. Therefore, it has been widely used in heartbeat detection. The researchers use different levels of decomposition in DWT and assume that the QRS wave can be characterized by some detail coefficients based on its spectrum. Considering the sampling frequency of the ECG signals analyzed, the detail coefficients 1-4 (corresponding to frequencies of 11.25-180 Hz), 3-5 (corresponding to frequencies of 5.75-45 Hz), and 4 (corresponding to frequencies of 15.6-31.1 Hz) are usually selected in the existing studies for further analysis.

However, DWT has drawbacks, including high sensitivity to signal offset and reduced temporal resolution at coarse scales. Therefore, some studies have since focused on reducing or eliminating the offset variance of DWT. One of the methods commonly used in ECG denoising is the stationary wavelet transform (SWT). In SWT, the filter coefficients are up-sampling in each step instead of down-sampling the signals after filtering. As a result, the resulting coefficients have the same length as the original signals. Due to its redundant representation, SWT is invariant to translation. It is, thus, suitable for detecting the exact location of a targeting event in a signal such as an edge. However, SWT has a particular disadvantage of having a phase shift with respect to the original signal. This is due to the fact that most wavelet decomposition schemes use FIR filter bank and many of them do not even have a linear phase. This phase shift results in QRS wave complex being projected to different temporal locations with different levels of SWT detail coefficients, all of which are different from their locations in the original signal. Clearly, this phase shift is undesirable in heartbeat detection. Although the offset is limited to the millisecond order of magnitude, synchronization is an important issue in QRS detection applications, especially in real-time applications. In addition, since the phase shift follows the SWT scale, it is expected that the phase shift increases with the scale. As a result, the final detail coefficients and the final approximate coefficients will have the most phase shift compared to the original input signal. It may result in more information being lost at higher ranges. In noisy environments where it is more difficult to localize QRS wave complex, removing these phase shifts becomes a more sensitive issue. In the field of ECG data processing, there is a lack of methods to eliminate such phase shifts in the current state of the art, and thus all existing QRS detections are performed in only one layer of wavelet detail coefficients, while a large amount of useful information contained in the wavelet detail coefficients of other layers are not analyzed. This produces unfavorable images for both accuracy and robustness for detecting the QRS wave complex.

On the other hand, some studies have focused on the design of ECG devices with non-standard ECG lead configurations such as the upper arm, to enhance the wearability, convenience, reusability, and the possibility of using the devices for home measurements. However, the surface electrophysiological signals acquired by these devices through non-clinical standard locations contain not only ECG signals but also electromyography (EMG) signals. Therefore, one of the main challenges in the study of these devices is to improve the quality of acquisition of ECG signals in the presence of EMG artifacts interfering with the energy more than the geodesic of ECG signals. Due to the presence of EMG artifacts, phase-shift-free feature extraction and analysis is crucial in heartbeat detection compared to ECG signals. Given that SWT is widely used in these scenarios, eliminating the resulting phase shift becomes even more important.

Considering the importance of minimizing the phase shift, Daubechies formulated approximate zero-phase filters that are widely used in DWT (Ingrid Daubechies, "Ten Lectures on Wavelets," 1992). However, these filters cannot achieve exact zero-phase. Percival introduced a zero-phase wavelet called the Zefret transform to solve this problem (D. Percival, "Discrete Wavelet Transforms Based on Zero-Phase Daubechies Filters."). Later, Lenis et al. proposed an improved version of the SWT to address the problem of phase-free transform properties (Biomed. Eng. / Biomed. Tech., vol. 61, no. 1, pp. 37-56, Feb. 2016). While applying the standard SWT to the signals, they inverted the signals and applied the SWT to the generated sequence. The transform is then inverted again and the transformed signals are summed at each scale. The resulting coefficients have zero phase shift. Thus, this method claims to be able to detect the beginning of a P-wave with very high accuracy. Eliminating the phase shift in higher scales provides the opportunity to combine information from different scales for more accurate heartbeat detection. However, these existing methods that still suffer from processing of high noise data are not designed for ECG, and applying them to ECG data suffers from poor processing, as well as poor robustness, and poor applicability to data acquired by ECG devices with non-standard ECG lead configurations such as the upper arm.

## SUMMARY

In view of the disadvantages in the prior art, the present disclosure provides a method and a system for processing surface electrophysiological signals to eliminate the phase shifts of the signals. It is intended to process ECG signals by combining a SWT-based zero-phase filter bank and a voting strategy for different SWT scales to improve the processing capability of noise and the robustness of the method, making the method more applicable to data acquired by ECG devices with non-standard ECG lead configurations, such as the upper arm.

A method for processing surface electrophysiological signals to eliminate signal phase shifts, comprising steps of:

step 1, acquiring surface electrophysiological signals from a human skin surface;
step 2, processing the surface electrophysiological signals using a zero-phase filter based on a stationary wavelet transform, to obtain a number of layers of wavelet detail coefficients and approximate coefficients;
step 3, detecting a QRS wave complex for each layer of wavelet detail coefficients, and obtaining a heartbeat detection result from each layer of wavelet detail coefficients by a weighted voting scheme.

Preferably, the surface electrophysiological signals are acquired by:

1) acquiring from standard electrode locations specified by clinical electrocardiogram (ECG) signals; or
2) acquiring from non-clinical standard locations.

Preferably, the non-clinical standard locations comprise an upper arm, a wrist, behind an ear, inside an ear, a hip, two legs, and two feet.

Preferably, step 2 specifically comprises steps of:

step 2.1, taking a second-order Daubechies wavelet transform as a mother wavelet and decomposing the mother wavelet and 4-7 layers, to obtain 4-7 wavelet detail coefficients and 1 approximate coefficient;
step 2.2, removing the phase shifts when decomposing each layer of wavelet using steps of:

1) filtering firstly by standard wavelets with a formula:

$$X(z)H(z) = D(z)$$

where z is a z-transform operator, X(z) is a z-transform of the signals, H(z) is a wavelet detail function of the layer, and D(z) is a coefficient of the layer;

2) operating a time-reversal sequence, with an equivalent formula in a z-transform domain:

$$D(\frac{1}{z}) = X(\frac{1}{z}) \; H(\frac{1}{z}) \; ;$$

3) filtering again on an output of a second step identical to a first step:

$$D(\frac{1}{z}) \; H(z) = X(\frac{1}{z}) \; H(\frac{1}{z}) \; H(z) \; ;$$

4) operating a time-reversal sequence on an output of a third step:

$$D`(z)=X(z)\ H(z)\ H(\frac{1}{z})\ ;$$

where D'(z) is a last zero-phase-shift wavelet detail coefficient of the layer; and then
operating same on the approximate coefficients by a wavelet generalization function G(z) of the layer.

Preferably, the wavelet detail coefficients are 5 layers, and frequency contents of the 5 layers of wavelet detail coefficients are 64-128 Hz, 32-64 Hz, 16-32 Hz, 8-16 Hz and 4-8 Hz, respectively.

Preferably, in step 3, a Pan-Tompkins algorithm is adopted for detecting the QRS wave complex.

Preferably, in step 3, the voting scheme comprises:

a result of each layer of detail coefficients detecting the QRS wave complex gets a corresponding weight of a voting coefficient, and a final result weighted by all layers of voting coefficients is a final QRS detection result; the voting comprises:
using a moving window of length 200 ms on a sequence of detected heartbeats;
detecting the heartbeats if a result of weight coefficients in the window exceeds a preset threshold;
setting a location where the heartbeats are detected to be an average of a temporal location of all positive votes;
combining at least two heartbeats into one heartbeat by time averaging if the at least two heartbeats are detected in a time interval of less than 200 ms.

The present disclosure further provides a system for implementing the method for processing surface electrophysiological signals, comprising:

an input module configured for inputting surface electrophysiological signal data;
a feature extraction module configured for processing the surface electrophysiological signal data using the zero-phase filter based on the stationary wavelet transform, to obtain 4-7 layers of wavelet detail coefficients and 1 approximate coefficients;
a voting module configured for detecting the QRS wave complex for all wavelet detail coefficients, and obtaining the heartbeat detection results from all wavelet detail coefficients by the weighted voting scheme.

The present disclosure further provides a device for detecting heartbeats, comprising a device for detecting the surface electrophysiological signals and the system described above.

The present disclosure further provides a computer-readable storage medium storing thereon a computer program configured for implementing the method for processing the surface electrophysiological signals described above.

The "surface electrophysiological signals" of the present disclosure comprise the signals (ECG signals) exhibiting the cardiac electrical activity at these locations, and also comprise signals from other sources such as skeletal muscle electrical signals, smooth muscle electrical signals and neuronal electrical signals.

The present disclosure provides a combination of a SWT-based zero-phase filter bank and a voting strategy for different SWT scales to achieve accurate extraction of electrophysiological signal components with fixed time-domain waveform characteristics, such as QRS wave complex in ECG signals, from the surface electrophysiological signals, without causing distortion of waveforms of the extracted signals due to the phase shifts. The method of the present disclosure increases the robustness of the noise. Experiments have demonstrated that the method of the present disclosure performs better than the prior art in terms of both sensitivity and positive predictive value. Therefore, the present disclosure is capable of accurately detecting QRS in high-noise environments (e.g., with high skeletal muscle electrical signals, smooth muscle electrical signals, and neuron electrical signals), and is highly applicable to application scenarios such as upper arm detection of electrocardiograms, which has a good application prospect.

Obviously, according to the above contents of the present disclosure, according to the ordinary technical knowledge and customary means in the field, without departing from the above basic technical concepts of the present disclosure, other various forms of modifications, substitutions or changes can be made.

The following specific implementations in the form of embodiments will further explain the above contents of the present disclosure in detail. However, it should not be understood that the scope of the above subject matter of the present disclosure is limited to the following examples. Any technology realized on the basis of the above contents of the present disclosure is within the scope of the present disclosure.

## BRIEF DESCRIPTION OF DRAWINGS

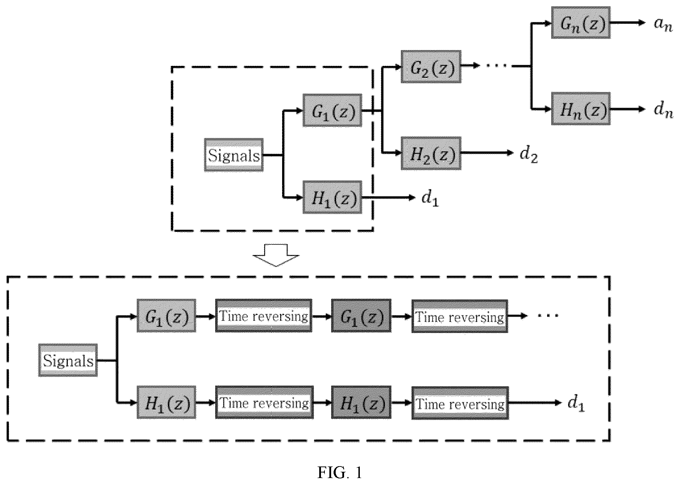

FIG. 1 shows a block diagram of a SWT-based zero-phase filter bank;

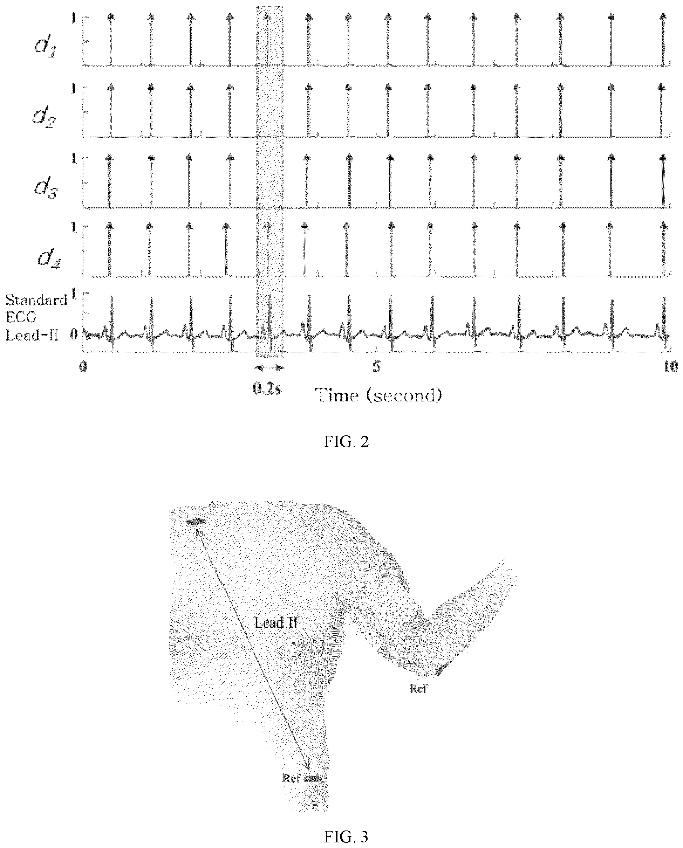

FIG. 2 shows a voting scheme. Pan-Tompkins outputs applied to different detail coefficients $d_i$ are shown. An example of a moving window is shown as a pink rectangle. In this window, if only $d_3$ or $d_2$ are considered, the data for this heartbeat will be lost. However, the system prevents such information from being lost.

FIG. 3 shows electrode locations in a data detection experimental paradigm designed to validate the zero-phase surface electrophysiological signaling system: three high-density electrode arrays containing 64 electrodes each (yellow) are placed in the left upper arm, and the reference electrode is placed at the elbow. Lead II channels of standard ECG are acquired simultaneously: a signal electrode is located at LA (right upper shoulder) and reference electrode RL (right waist).

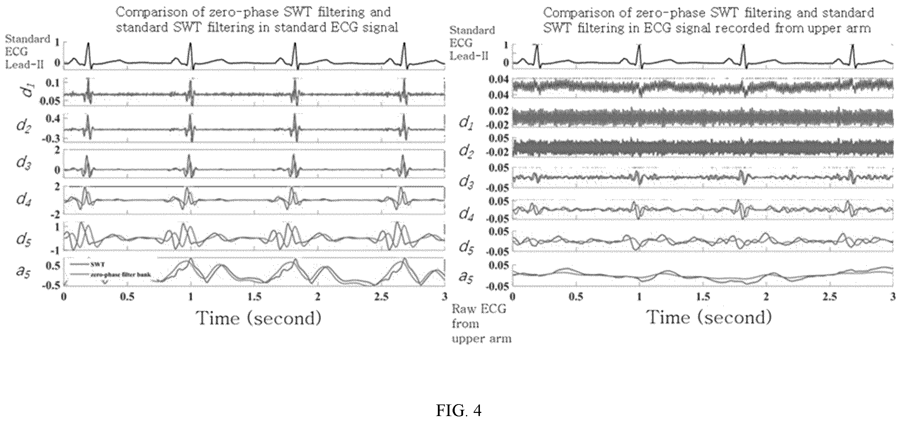

FIG. 4 shows the detail coefficients and approximate coefficients of applying SWT and zero-phase filter bank to the standard ECG lead II signal (left) and a typical upper arm (right) signal, respectively. A significant phase difference is presented between the different layers for SWT, while no corresponding phase difference exists on the side of the zero-phase filter bank.

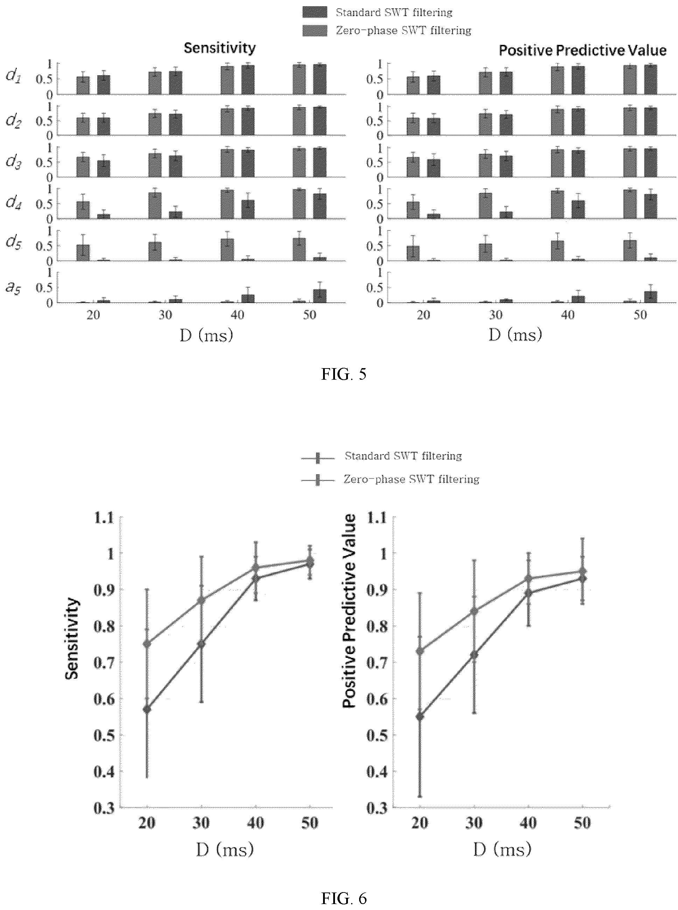

FIG. 5 shows the performance of the SWT-based zero-phase filter bank and the conventional SWT in detecting heartbeats from the upper arm signal before voting (i.e., using only the respective detail and approximate coefficients): sensitivity (left) and positive predictive value (right). The horizontal axis D value is the maximum permissible difference (in milliseconds) between the detected Q-wave peak location and the Q-wave peak location in the second lead of the standard ECG. Error bars indicate the mean +/- standard deviation of SE (left) and PPV (right) across all subjects and trials. It is clear that the performance of the zero-phase filter bank is significantly higher than that of the conventional SWT method when the requirement for the Q-wave peak location is high (small D-value).

FIG. 6 shows the performance of the SWT-based zero-phase filter bank and the conventional SWT in detecting heartbeats from the upper arm signal after voting: sensitivity (left) and positive predictive value (right). The horizontal axis D value is the maximum permissible difference (in milliseconds) between the detected Q-wave peak location and the Q-wave peak location in the second lead of the standard ECG. Error bars indicate the mean +/standard deviation of SE (left) and PPV (right) across all subjects and trials. It is clear that the performance of the zero-phase filter bank is significantly higher than that of the conventional SWT method when the requirement for the Q-wave peak location is high (small D value). Also, the performance is significantly improved compared to the results in FIG. 5 (before voting).

## DESCRIPTION OF EMBODIMENTS

It should be particularly noted that the algorithms implementing the steps of data acquisition, transmission, storage, and processing not specified in the embodiments, as well as the hardware structures, circuit connections, and the like not specified can be realized by what has been disclosed in the prior art.

Embodiment 1: Method and System for processing surface electrophysiological signals to eliminate signal phase shifts

The system according to the present disclosure comprises:

an input module configured for inputting the surface electrophysiological signal data; in some preferred embodiments, the surface electrophysiological signal data is acquired from the upper arm, wrist, behind the ear, or within the ear;

a feature extraction module configured for processing the surface electrophysiological signal data using a zero-phase filter based on a stationary wavelet transform, to obtain 4-7 layers of wavelet detail coefficients and 1 approximate coefficient;

a voting module configured for detecting a QRS wave complex for all wavelet detail coefficients, and obtaining the heartbeat detection results from all wavelet detail coefficients by a weighted voting scheme.

The method for processing the surface electrophysiological signals using the above system specifically includes steps of:

step 1, acquiring surface electrophysiological signals from a human skin surface;

step 2, processing the surface electrophysiological signal data using a zero-phase filter based on a stationary wavelet transform, to obtain a number of layers of wavelet detail coefficients and approximate coefficients;

Specifically step 2 includes steps of:

step 2.1, taking a second-order Daubechies wavelet transform as a mother wavelet and decomposing the mother wavelet and 4-7 layers, to obtain 4-7 wavelet detail coefficients and 1 approximate coefficient;

step 2.2, removing the phase shifts when decomposing each layer of wavelet using steps of:

1) filtering firstly by standard wavelets with a formula:

$$X(z)H(z) = D(z)$$

where z is a z-transform operator, X(z) is a z-transform of the signals, H(z) is a wavelet detail function of the layer, and D(z) is a coefficient of the layer;

2) operating a time-reversal sequence, with an equivalent formula in a z-transform domain:

$$D(\frac{1}{z})=X(\frac{1}{z})\ H(\frac{1}{z})\ ;$$

3) filtering again on an output of a second step identical to a first step:

$$D(\frac{1}{z})\ H(z)=X(\frac{1}{z})\ H(\frac{1}{z})\ H(z)\ ;$$

4) operating a time-reversal sequence on an output of a third step:

$$D`(z)=X(z)\ H(z)\ H(\frac{1}{z})\ ;$$

where D'(z) is a last zero-phase-shift wavelet detail coefficient (a.d) of the layer; and then

operating same on the approximate coefficients by a wavelet generalization function G(z) for the layer.

step 3, detecting a QRS wave complex for each layer of wavelet detail coefficients, and obtaining a heartbeat detection result from each layer of wavelet detail coefficients by a weighted voting scheme.

The details of the voting scheme are as follows:

the detection results of all QRS wave detail coefficients are made to obtain an equal weight vote, the vote is configured to determine whether or not a heartbeat is detected;

each layer of detail coefficients detecting the QRS wave complex results get a corresponding weight of a voting coefficient, and a final result weighted by all layers of voting coefficients is a final QRS detection result; the voting comprises:

using a moving window of length 200 ms on a sequence of detected heartbeats;

detecting the heartbeat if a result of weight coefficients in the window exceeds a preset threshold;

setting a location where the heartbeat is detected to be an average of a temporal location of all positive votes; where the weight coefficients can be set according to the equal weight method, or according to the information contained in the wavelet detail coefficients of each layer.

combining at least two heartbeats into one heartbeat by time averaging if the at least two heartbeats are detected in a time interval of less than 200 ms.

The technical solution of the present disclosure is further described in the following by means of experiments, and the unspecified steps are the same as in Embodiment 1.

EXPERIMENTAL Embodiment 1: detecting heartbeats from a human upper arm by SWT-based zero-phase filter bank combined with a voting strategy

i. Experimental method

1. Description of Dataset

The dataset for this study is taken from 9 participants (all male, 24-34 years old), with a duration of 5 minutes, each recording a total of 193 (64*3+1) channels of data. All participants are free of heart disease. As shown in FIG. 3, the electrodes consisted of one channel located on the right shoulder and three high-density 64-channel grids placed around the upper arm. The multichannel grids are equally spaced from each other, and the reference point is located at the elbow. The reference point for the right shoulder electrode is placed at the left hip, corresponding to standard ECG lead II. Before the experiment began, each participant signs a written informed consent form. The experiment is approved by the Office of Ethics Research at the University of Waterloo.

Each participant is asked to sit in a comfortable chair with his/her left arm in a resting location on the chair. Data are then recorded simultaneously from 193 channels using an EMG-USB2+ biosignal amplifier and a hardware bandpass filter from 0.1 Hz to 500 Hz. These recordings are sampled at a frequency of 2048 Hz. The data are then low-pass filtered using a Butterworth filter with a cutoff frequency of 100 Hz. Next, the data is down-sampled, and the sampling frequency is reduced to 256 Hz.

The data of this experimental example are acquired from the upper arm, belonging to the scenario of extracting the ECG activity in a higher noise environment.

2. Data Processing

Experimental group: the method and system adopted in this part are the same as in Embodiment 1.

Comparison group: the standard stationary wavelet transform (SWT) in the prior art is adopted.

3. Evaluation and Statistical Analysis of Sensitivity (SE) and Positive Predictive Value (PPV)

The formula is listed below:

$$SE = \frac{TP}{TP + FN} ;$$

$$PPV = \frac{TP}{TP + FP} ;$$

where TP is true positive and is the number of heartbeats detected in the range of intervals where the actual heartbeat is less than a predefined time interval (from lead II ECG). This time interval (D) takes values from 50 ms to 20 ms with a duration of 10 ms. FN is false negative and indicates the number of heartbeats that are not detected (or are missed), and FP indicates the number of heartbeats that are incorrectly detected.

The ability of the algorithm to detect heartbeats is indicated by SE. On the other hand, PPV demonstrates the accuracy of detection.

Each participant's data (240 seconds) is divided into twelve 20-second trials. Therefore a t-test is used to compare the performance vectors (SE and PPV) of the two algorithms with a length of 96 ($8 \times 12 = 96$) across conditions.

ii. Experimental Results

As shown in FIG. 4, the left figure shows the results of applying the original SWT and the zero-phase filter bank of the processing result of step 3 of Embodiment 1 to the ECG, and in the left figure, the details and approximate coefficients of the SWT when it is applied to the ECG signals, which are compared with the coefficients of the zero-phase filter bank based on the SWT. As can be seen from the figure, this filter bank prevents loss of information due to zero delay, especially at higher levels where cumulative delay is present. The same comparison is then performed for selected channels on the upper arm in the right column of FIG. 4. The results of the comparison show that after the processing of step 3 in Embodiment 1, the delays are eliminated and all wavelet detail coefficient scales are synchronized with the ECG.

Statistical analysis of SE and PPV is performed:

Before applying the voting scheme, the performance of the zero-phase filter bank used in step 3 in Embodiment 1 is compared to the original SWT. The results for all scales and 4 time intervals (D=20, 30, 40, 50 ms) are shown in FIG. 5. It can be seen that the zero-phase filter bank has a much higher performance than the SWT on the fourth and fifth scales for all time intervals. In the more challenging case, the difference is more pronounced when D becomes lower.

When D = 50 ms, d4 leads to the SE equal to 0.82 and 0.97 for the SWT and the zero-phase filter bank, respectively, and this performance decreases as D decreases. At the most challenging time interval (D=20 ms), the SE becomes 0.14 vs. 0.56, which is better for the zero-phase filter bank. On the other hand, when D=50 ms, d5 is already significantly superior for the zero-phase filter bank (0.11 vs. 0.74). When D = 20 ms, this difference becomes 0.02 vs. 0.48. This means that in the most challenging case, almost no heartbeats are detected in the last detail coefficients of the SWT, while almost half of the heartbeats are detected from the zero-phase filter bank. For d1 to d3, no significant difference exists when D is set to 40 ms and 50 ms. However, the zero-phase filter bank had a significant advantage in performance for d3 when D is set to 20 ms and 30 ms. The PPV also follows the same trend as the SE in all time intervals.

FIG. 6 shows the results of the voting scheme using d1 to d4 of the SWT and the zero-phase filter bank. The zero-phase filter bank has significantly higher SE and PPV in all Ds (p<0.05 for D=50 ms and p<0.01 for D=20, 30, and 40 ms). This shows that the zero-phase filter bank is more robust to reducing the acceptable interval from heartbeat in detection applications.

Comparing the results of the voting scheme in the zero-phase filter bank with the best individual a.d before voting, a.d4 has a lower SE (0.98 vs. 0.97) but a higher PPV (0.95 vs. 0.96) when D = 50 ms. When D is set to 40 ms, the SE becomes 0.94 vs. 0.96 and the voting scheme performs better. The voting scheme also has an advantage in terms of PPV, providing a parameter of 0.93 vs. 0.94. When D = 30 ms, the voting scheme increases the SE (0.86 vs. 0.87) but decreases the PPV (0.84 vs. 0.85) compared to a.d4. The voting scheme has the greatest advantage when D is set to 20 ms. It significantly increases the SE (0.67 vs. 0.75) and the PPV (0.67 vs. 0.73) compared to a.d3.

As can be seen from the above experimental results, using the method of the present disclosure, the SE and PPV are $0.98 \pm 0.04$ and $0.95 \pm 0.09$, respectively, for the heartbeat detection at an interval of 50 ms; and when the intervals are 40, 30, and 20 ms, the SE is to $0.96 \pm 0.07$, $0.87 \pm 0.12$, and $0.75 \pm 0.15$, respectively. The PPV also follows the same trend, with PPV being to $0.94 \pm 0.07$, $0.84 \pm 0.14$ and $0.73 \pm 0.16$ at intervals of 40, 30 and 20 ms from the actual heartbeat, respectively. In addition, the method of the present disclosure outperforms the original SWT in terms of both SE and PPV. This demonstrates that the method of the present disclosure is highly accurate and robust in detecting high noise data.

As can be seen from the above embodiments and experimental examples, the method and system according to the present disclosure are capable of accurately detecting QRS in high noise environments, and are highly applicable to application scenarios such as upper-arm detection of electrocardiograms, and have good application prospects.

**Claims**

1. A method for processing surface electrophysiological signals to eliminate signal phase shifts, comprising steps of:

   step 1, acquiring surface electrophysiological signals from a human skin surface;
   step 2, processing the surface electrophysiological signals using a zero-phase filter based on a stationary wavelet transform, to obtain a number of layers of wavelet detail coefficients and approximate coefficients;
   step 3, detecting a QRS wave complex for each layer of wavelet detail coefficients, and obtaining a heartbeat detection result from each layer of wavelet detail coefficients by a weighted voting scheme.

2. The method for processing surface electrophysiological signals according to claim 1, wherein: the surface electrophysiological signals are acquired by:

   1) collecting from standard electrode locations specified by clinical electrocardiogram (ECG) signals; or
   2) collecting from non-clinical standard locations.

3. The method for processing surface electrophysiological signals according to claim 2, wherein: the non-clinical standard locations comprise an upper arm, a wrist, behind an ear, inside an ear, a hip, two legs, and two feet.

4. The method for processing surface electrophysiological signals according to claim 1, wherein: step 2 specifically comprises steps of:

   step 2.1, taking a second-order Daubechies wavelet transform as a mother wavelet and decomposing the mother wavelet and 4-7 layers, to obtain 4-7 wavelet detail coefficients and 1 approximate coefficient;
   step 2.2, removing the phase shifts when decomposing each layer of wavelet using steps of:

      1) filtering firstly by standard wavelets with a formula:

$$X(z)H(z) = D(z)$$

where z is a z-transform operator, X(z) is a z-transform of the signals, H(z) is a wavelet detail function of the layer, and D(z) is a coefficient of the layer;

2) operating a time-reversal sequence, with an equivalent formula in a z-transform domain:

$$D(\frac{1}{z})=X(\frac{1}{z})\ H(\frac{1}{z})\ ;$$

3) filtering again on an output of a second step identical to a first step:

$$D(\frac{1}{z})\ H(z)=X(\frac{1}{z})\ H(\frac{1}{z})\ H(z)\ ;$$

4) operating a time-reversal sequence on an output of a third step:

$$D`(z)=X(z)\ H(z)\ H(\frac{1}{z})\ ;$$

where D'(z) is a last zero-phase-shift wavelet detail coefficient of the layer; and then
operating same on the approximate coefficients by a wavelet generalization function G(z) of the layer.

5. The method for processing surface electrophysiological signals according to claim 4, wherein: the wavelet detail coefficients are 5 layers, and frequency contents of the 5 layers of wavelet detail coefficients are 64-128 Hz, 32-64 Hz, 16-32 Hz, 8-16 Hz and 4-8 Hz, respectively.

6. The method for processing surface electrophysiological signals according to claim 1, wherein: in step 3, a Pan-Tompkins algorithm is adopted for detecting the QRS wave complex.

7. The method for processing surface electrophysiological signals according to claim 1, wherein: in step 3, the voting scheme comprises:
a result of each layer of detail coefficients detecting the QRS wave complex gets a corresponding weight of a voting coefficient, and a final result weighted by all layers of voting coefficients is a final QRS detection result; the voting comprises:

using a moving window of length 200 ms on a sequence of detected heartbeats;
detecting the heartbeats if a result of weight coefficients in the window exceeds a preset threshold;
setting a location where the heartbeats are detected to be an average of a temporal location of all positive votes;
combining at least two heartbeats into one heartbeat by time averaging if the at least two heartbeats are detected in a time interval of less than 200 ms.

8. A system for implementing the method for processing surface electrophysiological signals according to any of claims 1-7, comprising:

an input module configured for inputting surface electrophysiological signal data;
a feature extraction module configured for processing the surface electrophysiological signal data using the zero-phase filter based on the stationary wavelet transform, to obtain 4-7 layers of wavelet detail coefficients and 1 approximate coefficients;
a voting module configured for detecting the QRS wave complex for all wavelet detail coefficients, and obtaining the heartbeat detection results from all wavelet detail coefficients by the weighted voting scheme.

9. A device for detecting heartbeats, comprising a device for detecting surface electrophysiological signals and the system according to claim 8.

**10.** A computer-readable storage medium, storing thereon a computer program configured for implementing the method for processing the surface electrophysiological signals according to any of claims 1-7.

FIG. 1

FIG. 2

FIG. 3

Comparison of zero-phase SWT filtering and standard SWT filtering in standard ECG signal

Comparison of zero-phase SWT filtering and standard SWT filtering in ECG signal recorded from upper arm

FIG. 4

FIG. 5

FIG. 6

## INTERNATIONAL SEARCH REPORT

| International application No. |
|---|
| **PCT/CN2023/120071** |

### A. CLASSIFICATION OF SUBJECT MATTER

A61B 5/346(2021.01)i;  A61B 5/366(2021.01)i

According to International Patent Classification (IPC) or to both national classification and IPC

### B. FIELDS SEARCHED

Minimum documentation searched (classification system followed by classification symbols)

IPC:  A61B

Documentation searched other than minimum documentation to the extent that such documents are included in the fields searched

Electronic data base consulted during the international search (name of data base and, where practicable, search terms used)

CNTXT, WPABSC, ENTXTC, CJFD: 相移, 消除, 去除, 平稳, 稳定, 小波变换, 滤波, 零相位, 心电, 检测, 权重, 加权, 投票, eliminat+, signal, phase, shift, stationary, wavelet, transform, SWT, zero, filter, electrocardiogram, QRS, wave, detect+, weight, voting

### C. DOCUMENTS CONSIDERED TO BE RELEVANT

| Category* | Citation of document, with indication, where appropriate, of the relevant passages | Relevant to claim No. |
|---|---|---|
| PX | CN 115486856 A (JIANG NING et al.) 20 December 2022 (2022-12-20)<br>claims 1-10 | 1-10 |
| A | CN 105741305 A (SHENZHEN IKINLOOP TECHNOLOGY CO., LTD.) 06 July 2016 (2016-07-06)<br>description, paragraphs [0003]-[0086], and figures 1-4 | 1-10 |
| A | CN 107951485 A (LEPU MEDICAL EQUIPMENT (BEIJING) CO., LTD.) 24 April 2018 (2018-04-24)<br>entire document | 1-10 |
| A | CN 114027853 A (ANHUI HEARTVOICE MEDICAL TECHNOLOGY CO., LTD.) 11 February 2022 (2022-02-11)<br>entire document | 1-10 |
| A | CN 114648040 A (HAN HONGGUANG et al.) 21 June 2022 (2022-06-21)<br>entire document | 1-10 |

☑ Further documents are listed in the continuation of Box C.    ☑ See patent family annex.

| | |
|---|---|
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **13 December 2023** | **20 December 2023** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/120071**

### C.     DOCUMENTS CONSIDERED TO BE RELEVANT

| Category* | Citation of document, with indication, where appropriate, of the relevant passages | Relevant to claim No. |
|---|---|---|
| A | CN 106805965 A (SHENZHEN INSTITUTE OF ADVANCED TECHNOLOGY) 09 June 2017 (2017-06-09)<br>         entire document | 1-10 |
| A | US 2012123232 A1 (NAJARIAN, Kayvan et al.) 17 May 2012 (2012-05-17)<br>         entire document | 1-10 |

Form PCT/ISA/210 (second sheet) (July 2022)

## INTERNATIONAL SEARCH REPORT
### Information on patent family members

International application No.

**PCT/CN2023/120071**

| Patent document cited in search report | | | Publication date (day/month/year) | Patent family member(s) | | | Publication date (day/month/year) |
|---|---|---|---|---|---|---|---|
| CN | 115486856 | A | 20 December 2022 | None | | | |
| CN | 105741305 | A | 06 July 2016 | WO | 2017148451 | A1 | 08 September 2017 |
| CN | 107951485 | A | 24 April 2018 | EP | 3692904 | A1 | 12 August 2020 |
| | | | | US | 2020260980 | A1 | 20 August 2020 |
| | | | | US | 11234629 | B2 | 01 February 2022 |
| | | | | WO | 2019100565 | A1 | 31 May 2019 |
| CN | 114027853 | A | 11 February 2022 | None | | | |
| CN | 114648040 | A | 21 June 2022 | None | | | |
| CN | 106805965 | A | 09 June 2017 | None | | | |
| US | 2012123232 | A1 | 17 May 2012 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **INGRID DAUBECHIES**. *Ten Lectures on Wavelets*, 1992
- **D. PERCIVAL**. *Discrete Wavelet Transforms Based on Zero-Phase Daubechies Filters*
- *Biomed. Eng. / Biomed. Tech.*, February 2016, vol. 61 (1), 37-56